(19) Europäisches Patentamt
European Patent Office
Office européen des brevets

(11) Veröffentlichungsnummer : **0 424 738 B1**

(12) **EUROPÄISCHE PATENTSCHRIFT**

(45) Veröffentlichungstag der Patentschrift :
**26.01.94 Patentblatt 94/04**

(51) Int. Cl.$^5$ : **C07C 209/48,** C07C 211/13, C07D 211/26

(21) Anmeldenummer : **90119476.1**

(22) Anmeldetag : **11.10.90**

# (54) Verfahren zur Herstellung von Aminen.

(30) Priorität : **21.10.89 DE 3935112**

(43) Veröffentlichungstag der Anmeldung :
**02.05.91 Patentblatt 91/18**

(45) Bekanntmachung des Hinweises auf die Patenterteilung :
**26.01.94 Patentblatt 94/04**

(84) Benannte Vertragsstaaten :
**BE CH DE ES FR GB IT LI NL**

(56) Entgegenhaltungen :
**US-A- 3 246 000**
**PATENT ABSTRACTS OF JAPAN, Band 12, Nr. 159 (C-495)[3006], 14. Mai 1988; & JP-A-62 270 550**
**CHEMICAL ABSTRACTS, Band 91, Nr. 3, 16. Juli 1979, page 598, Zusammenfassung Nr. 19880n, Columbus, Ohio, US; & JP-A-79 05 903**

(73) Patentinhaber : **BASF Aktiengesellschaft**
**Carl-Bosch-Strasse 38**
**D-67063 Ludwigshafen (DE)**

(72) Erfinder : **Koehler, Ulrich, Dr.**
**Werderstrasse 48**
**W-6900 Heidelberg (DE)**
Erfinder : **Siegel, Hardo, Dr.**
**Hans-Purrmann-Allee 25**
**W-6720 Speyer (DE)**
Erfinder : **Jaeger, Peter, Dr.**
**Panoramastrasse 14**
**W-6719 Battenberg (DE)**
Erfinder : **Irgang, Matthias, Dr.**
**Andreas-Hofer-Weg 41**
**W-6900 Heidelberg (DE)**

**EP 0 424 738 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Diese Erfindung betrifft ein neues Verfahren zur Herstellung der Amine 4-Aminomethyl-1,8-diaminooctan und/oder 3-(4-Aminobutyl)-piperidin durch katalytische Hydrierung von 1,3,6-Tricyanohexan.

4-Aminomethyl-1,8-diaminooctan und 3-(4-Aminobutyl)-piperidin sind wertvolle Zwischenprodukte. So wird 4-Aminomethyl-1,8-diaminooctan z. B. als Härter für Epoxide oder für die Herstellung von Pestiziden verwendet, während 3-(4-Aminobutyl)-piperidin für die Herstellung von Nicotinsäure Interesse gefunden hat.

Es ist bekannt, daß 4-Aminomethyl-1,8-diaminooctan und 3-(4-Aminobutyl)-piperidin durch katalytische Hydrierung von 1,3,6-Tricyanohexan hergestellt werden können. So entstehen z.B. durch Hydrierung von 1,3,6-Tricyanohexan an Raney-Kobalt Gemische aus 4-Aminomethyl-1,8-diaminooctan und 3-(4-Aminobutyl)-piperidin. Bei diesem in der US-A-3,246,000 beschriebenen Verfahren ist der Anteil an undestillierbarem Rückstand recht hoch. Er beträgt nach Beispiel 2 der Patentschrift etwa 23 %. Eine selektive Herstellung von 4-Aminomethyl-1,8-diaminooctan und 3-(4-Amiobutyl)-piperidin ist nicht möglich. Günstigere Ergebnisse werden bei der in der JP 62/270-550 beschriebenen Hydrierung an Raney-Kobalt in Gegenwart von Wasser erzielt. Man erhält dabei 4-Aminomethyl-1,8-diaminooctan in ca. 70 % Ausbeute. Wird 1,2,6-Tricyanohexan nach den Angaben der JP 62/273 938 an Raney-Nickel in Gegenwart von Alkalihydroxid in Alkohol hydriert, so wird 4-Aminomethyl-1,8-diaminooctan in einer Ausbeute von 92 % erhalten.

Die beiden zuletzt genannten Verfahren weisen jedoch den Nachteil auf, daß die wahlweise bevorzugte Herstellung von 4-Aminomethyl-1,8-diaminooctan oder 3-(4-Aminobutyl)-piperidin nicht möglich ist. Auch sind die Ausbeuten noch verbesserungsbedürftig und die Verfahren eher zur diskontinuierlichen Fahrweise geeignet.

Es bestand deshalb die Aufgabe, ein Verfahren zu finden, das es gestattet, 4-Aminomethyl-1,8-diamonooctan und/oder 3-(4-Aminobutyl)-piperidin, vorteilhafter und auf besonders wirtschaftliche Weise herzustellen. Das neue Verfahren sollte außerdem eine wahlweise Herstellung der beiden Amine ermöglichen. Dabei war ein kontinuierliches Verfahren anzustreben. Die Katalysatoren sollten billige Nichtedelmetalle enthalten und eine lange Standzeit aufweisen.

Nach dem erfindungsgemäßen Verfahren, das diese Forderungen weitgehend erfüllt, werden die Amine der Formel

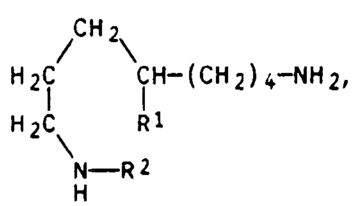

in der $R^1$ den $H_2N\text{-}CH_2$-Rest, $R^2$ ein Wasserstoffatom oder $R^1$ zusammen mit $R^2$ das Brückenglied $-CH_2-$ bedeuten, durch Umsetzung von 1,3,6-Tricyanohexan bei höheren Temperaturen und Drücken mit Wasserstoff an einem Katalysator der Kobaltoxid enthält, dadurch hergestellt, daß man einen Katalysator verwendet, der neben Kobaltoxid ein Oxid der Alkalimetalle, der Erdalkalimetalle, der Seltenen Erden oder der Metalle Scandium oder Yttrium enthält.

Nach dem neuen Verfahren hydriert man z.B. bei Temperaturen von 30 bis 250°C und Drücken von 50 bis 350 bar, wobei man einen Katalysator verwendet, der Kobaltoxid und ein Oxid der Alkalimetalle, der Erdalkalimetalle, der Seltenen Erden oder der Metalle Scandium oder Yttrium enthält. Als Oxide der Alkali- oder Erdalkalimetalle kommen z.B. $LiO_2$, $Na_2O$, $K_2O$, MgO, CaO, SrO und BaO in Betracht. Als Oxide der Seltenen Erden verwendet man z.B. $La_2O_3$, $Ce_2O_3$, $Gd_2O_3$, $Dy_2O_3$, $Sm_2O_3$, $Nd_2O_3$ oder $Er_2O_3$. Die katalytisch aktive Masse enthält Kobaltoxid als Hauptbestandteil und als Nebenbestandteil mindestens eines der genannten Metalloxide. Beispielsweise hat die katalytisch aktive Masse einen Gehalt an Kobaltoxid von 20 bis 95 Gew.-% und an den genannten Oxiden von 0,5 bis 20 Gew.-%. Bevorzugt enthalten die Katalysatoren neben Kobaltoxid und den Oxiden der Alkalimetalle, der Erdalkalimetalle, der Seltenen Erden oder der Metalle Scandium oder Yttrium noch mindestens ein Oxid der Elemente Eisen, Nickel, Mangan, Chrom, Molybdän, Wolfram oder Phosphor, von denen Eisen, Nickel und Mangan bevorzugt sind.

Besonders geeignete Katalysatoren sind z. B. solche, deren katalytisch aktive Masse mindestens zu 20 Gew.-% aus Kobaltoxid, höchstens zu 20 Gew.-% aus einem Oxid der Alkalimetalle, der Erdalkalimetalle, der Seltenen Erden oder der Metalle Scandium oder Yttrium und bis zu 60 Gew.-% aus einem Oxid der Elemente Eisen, Nickel, Mangan, Chrom, Molybdän, Wolfram oder Phosphor besteht. Das sind z.B. Katalysatoren, deren katalytisch aktive Masse zu 20 bis 95, vorzugsweise 40 bis 90 Gew.-% aus Kobaltoxid, zu 0,5 bis 20 Gew.-%

aus einem Oxid der Alkali- oder Erdalkalimetalle, der Seltenen Erden oder der Metalle Scandium oder Yttrium und zu 0,5 bis 60, vorzugsweise 1 bis 40 Gew.-% aus einem Oxid der Metalle Mangan, Nickel oder Eisen besteht.

Die Katalysatoren können als Vollkatalysatoren, z.B. in Form von Pulver oder Granulat, oder auch aufgetragen auf inerte Träger, beispielsweise Siliciumdioxid, Aluminiumoxid, Zeolithe, Titandioxid, Magnesiumoxid, Bims, Zirkonoxid oder Kohlenstoff verwendet werden. Der Katalysator, der die metallischen Bestandteile in Form ihrer Oxide enthält, wird in der Regel vor seinem Einsatz durch eine Wasserstoffbehandlung aktiviert, wobei die Oxide teilweise zu den Metallen reduziert werden.

Das 1,3,6-Tricyanohexan wird als Substanz oder als Lösung in einem Lösungsmittel eingesetzt. Geeignete Lösungsmittel sind z.B. Ether, wie Tetrahydrofuran, oder Dioxan, Alkohole, wie Methanol oder Ethanol oder andere für Hydrierungen an sich üblichen Lösungsmittel, wie Dimethylformamid oder N-Methyl-pyrrolidon.

Die Hydrierung wird diskontinuierlich, vorzugsweise aber kontinuierlich am Festbett (Riesel- oder Sumpffahrweise) oder in Suspension durchgeführt. Bei diskontinuierlichem Betrieb verfährt man z. B. so, daß man 1,3,6-Tricyanohexan oder dessen Lösung zusammen mit dem Katalysator in einen Hochdruckautoklaven gibt, Ammoniak und Wasserstoff aufpreßt und das Reaktionsgemisch erhitzt. Nach Ende der Reaktion wird abgekühlt, der Katalysator abgetrennt und das Reaktionsgemisch fraktioniert destilliert.

Der zur Hydrierung verwendete Wasserstoff wird im allgemeinen im größeren stöchiometrischen Überschuß verwendet. Er kann als Kreisgas in die Reaktion zurückgeführt werden. Der Wasserstoff kommt im allgemeinen technisch rein zum Einsatz. Beimengen von Inertgasen, z. B. Stickstoff, stören jedoch den Reaktionsablauf nicht.

Das erfindungsgemäße Verfahren erlaubt die wahlweise Herstellung eines der beiden Verfahrensprodukte. Will man bevorzugt 4-Aminomethyl-1,8-diaminooctan herstellen, so hydriert man bei möglichst niedrigen Temperaturen, das sind Temperaturen von 30 bis 120°C, vorzugsweise 50 bis 100°C. Unter diesen Bedingungen ist der Zusatz von Ammoniak im Verhältnis 1:1 bis 100:1 mol pro mol 1,3,6-Tricyanohexan vorteilhaft, um die Bildung von 3-(4-Aminobutyl)piperidin weiter zurückzudrängen.

Will man 3-(4-Aminobutyl)-piperidin herstellen, so hydriert man bei möglichst hohen Temperaturen, das sind Temperaturen von über 120 bis 250°C, vorzugsweise über 120 bis 200°C. In diesem Fall arbeitet man vorteilhafter mit wenig oder ohne Ammoniak (0 bis 3 mol $NH_3$) pro mol Trinitril. Man hydriert in beiden Fällen in dem oben angegebenen Druckbereich.

Das erfindungsgemäße Verfahren erlaubt es, das mengenmäßige Verhältnis der beiden Verfahrensprodukte 4-Aminomethyl-1,8-diaminooctan und 3-(4-Aminobutyl)-piperidin so zu variieren, daß man sie im Verhältnis 98:2 zu 2:98 erhält.

So werden beispielsweise bei einer Temperatur von 60°C, einem Druck von 300 bar $H_2$, einem 10fachen molaren Ammoniaküberschuß und einer Belastung von 0,05 kg/l·h (kontinuierlicher Betrieb) ein Reaktionsaustrag aus 97,9 Gew.-% 4-Aminomethyl-1,8-diaminooctan und 1,8 Gew.-% 3-(4-Aminobutyl)-piperidin erhalten, während bei 170°C ohne Ammoniak und sonst gleichen Bedingungen 1.8 Gew.-% 4-Aminomethyl-1,8-diaminooctan und 97.8 Gew.-% 3-(4-Aminobutyl)-piperidin erhalten werden (Beispiel 5). Nach dem neuen Verfahren erhält man die Verfahrensprodukte bei nahezu quantitativem Umsatz mit relativ hohen Raum-Zeit-Ausbeuten. Die Standzeiten (kontinuierliche Fahrweise) betragen > 2 Monate.

Die in nachstehenden Beispielen genannten Prozente sind Gewichtsprozente.

## Beispiele

### Beispiel 1

Herstellung geeigneter Katalysatoren

Katalysator A (Pulver)

Eine wäßrige Lösung, die Cobaltnitrat, Eisennitrat und Mangannitrat enthält und eine 30 %ige wäßrige Natriumbicarbonatlösung werden über getrennte Pumpen so in einen Rührbehälter dosiert, daß ein konstanter pH-Wert von 6.5 eingehalten wird. Dabei wird die Temperatur im Fällungsbehälter auf 50°C gehalten.

Das Fällungsprodukt wird auf eine Filterpresse gepumpt und Na-frei gewaschen. Anschließend vermischt man die Filterpaste in einem Mischer mit feingemahlenem Calciumhydroxid-Pulver, trocknet und calciniert das Produkt bei 500°C. Zum Schluß wird es auf eine Korngröße von 0,05 bis 0,2 mm gemahlen. Man erhält einen Katalysator mit der folgenden chemischen Zusammensetzung (glühverlustfrei berechnet):

65,2 Gew.-% CoO
4,7 Gew.-% $Mn_30_4$

10,0 Gew.-% CaO
20,1 Gew.-% $Fe_2O_3$

Katalysator B (Extrudate)

Aus einer wäßrigen Lösung die Cobaltnitrat, Eisennitrat, Nickelnitrat und Mangannitrat enthält, wird in gleicher Weise wie bei Katalysator A beschrieben, mit Sodalösung das Katalysatorvorprodukt ausgefällt, filtriert und ausgewaschen.

Die Filterpaste wird in einem Mischer mit Calciumhydroxid-Pulver (10% CaO bezogen auf die Summe der Oxide) gemischt, getrocknet und wiederum bei 500°C calciniert. Das so entstandene Produkt wird in einem Kneter verdichtet und in eine verformbare Konsistenz übergeführt. Das Material wird extrudiert, getrocknet und nochmals bei 650°C calciniert. Die 4-mm-Extrudate haben folgende chemische Zusammensetzung (glühverlustfrei berechnet):

69,2 Gew.-% CoO
4,9 Gew.-% $Mn_3O_4$
5,5 Gew.-% NiO
10,4 Gew.-% $Fe_2O_3$
10,0 Gew.-% CaO

Beispiel 2

Diskontinuierliche Hydrierung von 1,3,6-Tricyanohexan

20 g Tricyanohexan, 100 ml Tetrahydrofuran und 4 g des Katalysators A (s. Beispiel 1) werden in einem Autoklaven vorgelegt. Es wird mit Stickstoff gespült und 30 ml Ammoniak zugegeben. Man heizt auf 60°C auf und preßt 300 bar Wasserstoff auf, wobei stündlich Wasserstoff nachgepreßt wird. Nach Abkühlen, Entspannen, Filtrieren und Einengen werden durch Destillation 20,5 g 4-Aminomethyl-1,8-octandiamin erhalten (95,2 % Ausbeute).

Beispiel 3

Diskontinuierliche Hydrierung von 1,3,6-Tricyanohexan

Man verfährt wie in Beispiel 2 beschrieben, setzt jedoch einen Katalysator ein, der zu 65,3 % aus CoO, 5,2 % aus MnO, 10,3 % aus $Fe_2O_3$ und 19,2 % aus $La_2O_3$ besteht, und führt die Umsetzung bei 80°C durch. Es werden 21,3 g (98,9 %) 4-Aminomethyl-1,8-octandiamin erhalten.

Beispiel 4

Man verfährt wie im Beispiel 2 beschrieben, wobei man die Umsetzung jedoch bei 170°C und ohne Ammoniak durchführt. Es werden 16,9 g 3-(4-Aminobutyl)piperidin (87,9 % Ausbeute) erhalten.

Beispiel 5

Kontinuierliche Hydrierung von 1,3,6-Tricyanohexan

Ein Rieselreaktor mit der Länge von 3 m und einem Innendurchmesser von 16 mm wird mit dem Katalysator B (s. Beispiel 1) gefüllt. Am Kopfende des Reaktors wird eine 50 proz. Lösung von 1,3,6-Tricyanohexan in Tetrahydrofuran eingeleitet. Wasserstoff und Ammoniak werden ebenfalls am Kopfende eingeleitet. Bei einem stündlichen Zulauf von 500 ml/h der Tricyanohexan-Lösung, der unten angegebenen Menge Ammoniak und einem Wasserstoffdruck von 300 bar wird ein Umlauf von 9 l/h eingestellt. Es werden 3 Versuche bei unterschiedlichen Temperaturen durchgeführt. Die Austräge werden filtriert, eingeengt und destilliert. Man erhält die in der nachstehenden Tabelle angegebenen Massenanteile:

| Temp. (°C) | Zulauf $NH_3$ (ml/h) | 4-Aminomethyl-1,8-octandiamin (%) | 3-(4-Aminobutyl)-piperidin (%) | Rückstand (%) |
|---|---|---|---|---|
| 60 | 400 | 97,9 | 1,8 | 0,3 |
| 120 | 200 | 51,2 | 45,3 | 3,5 |
| 170 | 0 | 1,8 | 97,8 | 0,4 |

Beispiel 6

Kontinuierliche Hydrierung von 1,3,6-Tricyanohexan

Ein für die Sumpffahrweise geeigneter Reaktor mit einer Länge von 2 m und einem Durchmesser von 41 mm wird mit Katalysatorsträngen der Länge 7 m und dem Durchmesser von 4 mm gefüllt. Der Katalysator enthält die folgenden aktiven Bestandteile: 70,3 % CoO, 10,5 % NiO, 5,2 % MnO, 14,0 % $Na_2O$. Am unteren Ende des Reaktors wird eine 40 proz. Lösung von 1,3,6-Tricyanohexan in Methanol eingeleitet. Wasserstoff und Ammoniak werden ebenfalls von unten eingeleitet. Bei einem stündlichen Zulauf von 700 ml/h Tricyanohexan-Methanol-Lösung, 450 ml/h Ammoniak und einem Wasserstoffdruck von 300 bar wird ein Umlauf von 40 l/h eingestellt. Man arbeitet den Austrag wie in Beispiel 5 geschildert auf. Bei 80°C werden folgende Massenanteile erhalten: 4-Aminomethyl-1,8-octandiamin 93,7 %, 3-(4-Aminobutyl)-piperidin 5,3 %, Rückstand 1,0 %.

## Patentansprüche

1. Verfahren zur Herstellung der Amine der Formel

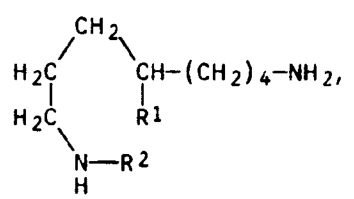

in der $R^1$ den $H_2N$-$CH_2$-Rest, $R^2$ ein Wasserstoffatom oder $R^1$ zusammen mit $R^2$ das Brückenglied -$CH_2$- bedeuten, durch Umsetzung von 1,3,6-Tricyanohexan bei höheren Temperaturen und Drücken mit Wasserstoff an einem Katalysator der Kobaltoxid enthält, dadurch gekennzeichnet, daß man einen Katalysator verwendet, der neben Kobaltoxid ein Oxid der Alkalimetalle, der Erdalkalimetalle, der Seltenen Erden oder der Metalle Scandium oder Yttrium enthält.

2. Verfahren nach Anspruch 1, dadurch gekennzeichnet, daß man einen Katalysator verwendet, der neben Kobaltoxid und einem Oxid der Alkalimetalle, der Erdalkalimetalle, der Seltenen Erden oder der Metalle Scandium oder Yttrium ein Oxid der Elemente Eisen, Nickel, Mangan, Chrom, Molybdän, Wolfram oder Phosphor enthält.

3. Verfahren nach Anspruch 1, dadurch gekennzeichnet, daß man einen Katalysator verwendet, der neben Kobaltoxid und einem Oxid der Alkalimetalle, der Erdalkalimetalle, der Seltenen Erden oder der Metalle Scandium oder Yttrium ein Oxid der Elemente Eisen, Nickel oder Mangan enthält.

4. Verfahren nach Anspruch 1, dadurch gekennzeichnet, daß man einen Katalysator verwendet, dessen katalytisch aktive Masse mindestens zu 20 Gew.-% aus Kobaltoxid, höchstens zu 20 Gew.-% aus einem Oxid der Alkalimetalle, der Erdalkalimetalle, der Seltenen Erden oder der Metalle Scandium oder Yttrium und bis zu 60 Gew.-% aus einem Oxid der Elemente Eisen, Nickel, Mangan, Chrom, Molybdän, Wolfram oder Phosphor besteht.

5. Verfahren nach Anspruch 1, dadurch gekennzeichnet, daß man einen Katalysator verwendet, dessen katalytisch aktive Masse zu 20 bis 95 Gew.-% aus Kobaltoxid, zu 0,5 bis 60 Gew.-% aus einem Oxid der

Metalle Mangan, Nickel oder Eisen und zu 0,5 bis 20 Gew.-% aus einem Oxid der Alkali- oder Erdalkalimetalle, der Seltenen Erden oder der Metalle Scandium oder Yttrium besteht.

6. Verfahren nach Anspruch 1, dadurch gekennzeichnet, daß man die katalytische Hydrierung bei Temperaturen von 30 bis 250°C und Drücken von 50 bis 350 bar durchführt.

7. Verfahren nach Anspruch 1, daß man die katalytische Hydrierung von 1,3,6-Tricyanohexan zur Herstellung von 4-Aminomethyl-1,8-diaminooctan bei Temperaturen von 30 bis 120°C durchführt.

8. Verfahren nach Anspruch 1, daß man die katalytische Hydrierung von 1,3,6-Tricyanohexan zur Herstellung von 3-(4-Aminobutyl)-piperidin bei Temperaturen von über 120 bis 250°C durchführt.

## Claims

1. A process for the preparation of amines of the formula

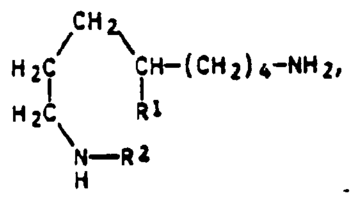

where $R^1$ is $H_2N\text{-}CH_2\text{-}$, and $R^2$ is a hydrogen atom, or $R^1$ and $R^2$ are a $\text{-}CH_2\text{-}$ bridge, by reacting 1,3,6-tricyanohexane with hydrogen at elevated temperature and pressure on a catalyst containing cobalt oxide, wherein a catalyst is used which, in addition to cobalt oxide, contains an oxide of the alkali metals, alkaline earth metals, rare earths or the metals scandium or yttrium.

2. A process as claimed in claim 1, wherein a catalyst is used which, in addition to cobalt oxide and an oxide of the alkali metals, alkaline earth metals, rare earths or the metals scandium or yttrium, contains an oxide of the elements iron, nickel, manganese, chromium, molybdenum, tungsten or phosphorus.

3. A process as claimed in claim 1, wherein a catalyst is used which, in addition to cobalt oxide and an oxide of the alkali metals, alkaline earth metals, rare earths or the metals scandium or yttrium, contains an oxide of the elements iron, nickel or manganese.

4. A process as claimed in claim 1, wherein a catalyst is used whose catalytically active material comprises at least 20% by weight of cobalt oxide, at most 20% by weight of an oxide of the alkali metals, alkaline earth metals, rare earths or the metals scandium or yttrium, and up to 60% by weight of an oxide of the elements iron, nickel, manganese, chromium, molybdenum, tungsten or phosphorus.

5. A process as claimed in claim 1, wherein a catalyst is used whose catalytically active material comprises from 20 to 95% by weight of cobalt oxide, from 0.5 to 60% by weight of an oxide of the metals manganese, nickel or iron, and from 0.5 to 20% by weight of an oxide of the alkali or alkaline earth metals, rare earths or the metals scandium or yttrium.

6. A process as claimed in claim 1, wherein the catalytic hydrogenation is carried out at from 30 to 250°C and at from 50 to 350 bar.

7. A process as claimed in claim 1, wherein the catalytic hydrogenation of 1,3,6-tricyanohexane for the preparation of 4-aminomethyl-1,8-diaminooctane is carried out at from 30 to 120°C.

8. A process as claimed in claim 1, wherein the catalytic hydrogenation of 1,3,6-tricyanohexane for the preparation of 3-(4-aminobutyl)piperidine is carried out at from above 120 to 250°C.

## Revendications

1. Procédé de préparation des amines de la formule suivante

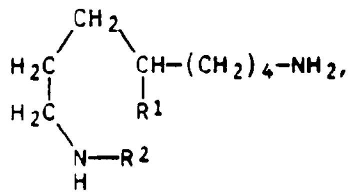

dans laquelle $R^1$ représente le radical $H_2N-CH_2-$, $R^2$ représente un atome d'hydrogène, ou bien $R^1$ et $R^2$ représentent ensemble l'élément de pontage $-CH_2-$, par la réaction du 1,3,6-tricyanohexane avec l'hydrogène sur un catalyseur qui contient de l'oxyde de cobalt, à des températures et des pressions élevées, caractérisé en ce que l'on utilise un catalyseur qui contient, outre de l'oxyde de cobalt, un oxyde des métaux alcalins, des métaux alcalino-terreux, des terres rares, ou des métaux scandium et ytrium.

2. Procédé suivant la revendication 1, caractérisé en ce que l'on utilise un catalyseur qui contient, outre de l'oxyde de cobalt et un oxyde des métaux alcalins, des métaux alcalino-terreux, des terres rares ou des métaux scandium ou ytrium, un oxyde des éléments fer, nickel, manganèse, chrome, molybdène, tungstène ou phosphore.

3. Procédé suivant la revendication 1, caractérisé en ce que l'on utilise un catalyseur qui contient, outre de l'oxyde de cobalt et un oxyde des métaux alcalins, des métaux alcalino-terreux, des terres rares ou des métaux scandium ou ytrium, un oxyde des éléments fer, nickel ou manganèse.

4. Procédé suivant la revendication 1, caractérisé en ce que l'on utilise un catalyseur dont la masse catalytiquement active est constituée par jusqu'au moins 20% en poids d'oxyde de cobalt, jusqu'au plus 20% en poids d'un oxyde des métaux alcalins, des métaux alcalino-terreux, des terres rares ou des métaux scandium ou ytrium et jusqu'à 60% en poids d'un oxyde des éléments fer, nickel, manganèse, chrome, molybdène, tungstène ou phosphore.

5. Procédé suivant la revendication 1, caractérisé en ce que l'on utilise un catalyseur dont la masse catalytiquement active se compose de jusqu'à 20 à 95% en poids d'oxyde de cobalt, de jusqu'à 0,5 à 60% en poids d'un oxyde des métaux manganése, nickel ou fer et de jusqu'à 0,5 à 20% en poids d'un oxyde des métaux alcalins ou des métaux alcalino-terreux, des terres rares ou des métaux scandium ou ytrium.

6. Procédé suivant la revendication 1, caractérisé en ce que l'on entreprend l'hydrogénation catalytique à des températures de 30 à 250°C et des pressions de 50 à 350 bars.

7. Procédé suivant la revendication 1, caractérisé en ce que l'on entreprend l'hydrogénation catalytique du 1,3,6-tricyanohexane à des températures de 30 à 120°C en vue de la préparation du 4-aminométhyl-1,8-diaminooctane.

8. Procédé suivant la revendication 1, caractérisé en ce que l'on entreprend l'hydrogénation catalytique du 1,3,6-tricyanohexane à des températures supérieures à 120°C-250°C en vue de la préparation de la 3-(4-aminobutyl)-pipéridine.